# EUROPEAN PATENT SPECIFICATION

(11) **EP 3 390 627 B1**
(45) Date of publication and mention of the grant of the patent: **23.09.2020**
(21) Application number: 16733346.7
(22) Date of filing: 17.06.2016
(51) Int. Cl.: C12N 9/32

(54) **METHOD FOR ENZYMATICALLY MODIFYING THE TRI-DIMENSIONAL STRUCTURE OF A PROTEIN**
VERFAHREN ZUR ENZYMATISCHEN MODIFIZIERUNG DER DREIDIMENSIONALEN STRUKTUR EINES PROTEINS
PROCÉDÉ DE MODIFICATION DE MANIÈRE ENZYMATIQUE DE LA STRUCTURE TRIDIMENSIONNELLE D'UNE PROTÉINE

(30) Priority: 14.12.2015 LU 92906
(43) Date of publication of application: 24.10.2018
(73) Proprietor: Luxembourg Institute of Science and Technology (LIST), 4362 Esch/Alzette (LU)
(72) Inventor: SERGEANT, Kjell, 6640 Vaux-sur-Sûre (BE)
(74) Representative: Lecomte & Partners
(86) International application number: PCT/EP2016/064094
(87) International publication number: WO 2017/102103

(56) References cited:
- RAO A GURURAJ: "The outlook for protein engineering in crop improvement", PLANT PHYSIOLOGY (ROCKVILLE), vol. 147, no. 1, May 2008 (2008-05), pages 6-12, XP002756483, ISSN: 0032-0889
- SIODLAK D ED - TSIKAS DIMITRIOS ET AL: "[alpha],[beta]-Dehydroamino acids in naturally occurring", AMINO ACIDS, SPRINGER VERLAG, AU, vol. 47, no. 1, 17 October 2014 (2014-10-17), pages 1-17, XP035418064, ISSN: 0939-4451, DOI: 10.1007/S00726-014-1846-4 [retrieved on 2014-10-17]
- GUPTA MADHVI ET AL: "De Novo Design of alpha,beta-Didehydrophenylalanine Containing Peptides: From Models to Applications", BIOPOLYMERS, vol. 95, no. 3, March 2011 (2011-03), pages 161-173, XP002756484,

## Description

### Technical field

The invention is directed to the field of stabilization of proteins in order to enhance their specificities and their activities. In particular, the invention is directed to the incorporation and use of modified amino acid residues in order to stabilize the structure of proteins. More particularly, the modified amino acid residue is didehydro-phenylalanine.

### Background art

Proteins are remarkably dynamic macromolecules, with conformational motions that play roles in all biological processes such as generating mechanical support, carrying out enzymatic reactions, and mediating signal transduction. Since the various states, more particularly the conformation of a protein molecule, may potentiate different functions there is considerable interest in the ability to generate proteins with a stable and predictable three dimensional structure.

The use of enzymes in industry and of proteins in general is still restricted in many cases. The greatest technical difficulty is the finding of suitable proteins which are stable under industrially desired conditions such as temperature, pH, requirements of activators, and/or the presence of inhibitors. The use of enzymatic reactions in the catalysis during industrial applications is thus limited by insufficient stability of the enzymes under the used reaction conditions or during purification.

Similarly, the use of protein therapeutics is hindered by the limited stability during long-term storage of the products.

US patent application published US 2013/281314 A1 relates to methods for screening for and using conformationally stabilized forms of a conformationally dynamic protein, such as a conformationally stabilized ubiquitin protein.

International patent application published WO 2011/073209 A1 refers to novel proteins, in particular hetero-multimeric proteins, capable of binding the extradomain B of fibronectin (ED-B). Furthermore, the disclosure refers to fusion proteins comprising said binding protein fused to a pharmaceutically and/or diagnostically active component. The disclosure is further directed to a method for the generation of such binding protein or fusion protein and to pharmaceutical/diagnostic compositions containing the same. In addition, the disclosure refers to libraries which are based on a scaffold protein comprising linear polyubiquitin chains with at least two interacting binding determining regions (BDR).

International patent application published WO 01/29247 A1 relates to cross-linking methods to stabilize polypeptides and polypeptide complexes for commercial uses (pharmaceutical, therapeutic, and industrial).

Several protein stabilization strategies are known in the art and are briefly reviewed here.

On the protein level, the most prominent approach is the discovery of stable biocatalysts from extremophilic and more particularly thermophilic, organisms, directed evolution, and computational and protein engineering. As most industrial enzymes are preferably used at elevated temperatures so that viscosity is reduced while reaction rates are increased industrial enzymes are often best derived from thermophilic microorganisms or extremophiles which resist to these extreme conditions.

For example, US patent application published US 8,592,192 relates to the field of stabilizing proteins, and more specifically to the field of stabilizing proteins without any modification of their primary sequence. Although enzymes of commercial relevance have been identified from them, this 'discovery' approach is limited by what can be found in nature. This approach has not yielded many commercially-relevant, thermostable biocatalysts as was initially hoped for and/or projected.

'Directed evolution' techniques are powerful approaches capable of generating stabilized enzymes, often also with altered/improved functional specificities. However, the approach is limited by the feasibility of the selection procedure.

Algorithms that calculate intra-molecular forces within proteins are being used to design and/or evolve enzymes with greater thermostability *in silico.* This approach is still severely hampered by the limited understanding of the intra-molecular forces and the processes involved in protein folding.

US patent application published US 2014/012777 relates to improved stabilization of polypeptides by incorporation of non-natural amino acids, such as hyper-hydrophobic amino acids, into the hydrophobic core regions of the polypeptides.

International patent application published WO 2008/085900 A2 relates to biomolecular engineering and design, including methods for the design and engineering of biopolymers such as proteins and nucleic acids.

US patent application published US 5,811,515 generally relates to the synthesis of conformationally restricted amino acids and peptides. More specifically, the invention relates to the synthesis of conformationally restricted amino acids and peptides by catalyzed ring closing metathesis ("RCM"). Other approaches, often referred to as protein engineering, such as derivatization (e.g. PEGylation, addition of polymeric sucrose and/or dextran, methoxypolyethylene glycol, *etc.)* and old methods of protein cross-linking (e.g. production of cross-linked enzyme crystals or CLEC's) can also be cited. Unfortunately, these approaches are often ineffectual or cause dramatic losses in activity.

European patent application EP 0355 039 A2 is linked to the field of protein engineering and provides methods for the production of proteins with modified stability, preferably towards thermal denaturation and/or chemical modification, by means of one or more amino acid replacements at specific sites in proteins using protein engineering techniques.

Other strategies, such as (a) catalyst immobilization or (b) use of organic solvents in the reaction medium (termed medium engineering) have been employed.

However, despite the great technological potential of catalyst immobilization, few large-scale processes utilize immobilized enzymes. Severe restrictions often arise in scale-up because of additional costs, activity losses, and issues regarding diffusion.

Regarding the medium engineering field, enhanced thermostability in organic media has proven to be an additional and significant bonus. It is hypothesized that partial or almost total substitution of water can be beneficial since water is involved in enzyme inactivation. Whatever the mechanism, numerous cases have recently been reported where remarkable enzyme stability has been obtained in organic media such as polyglycols and glymes. However, medium engineering is unlikely to solve all biocatalysis stability problems.

Molecular biological techniques have made it possible to stabilize some proteins by, e.g., engineering fusion-proteins. To make a fusion-protein, a single nucleic acid construct is created that directs the expression of modular domains derived from at least two proteins as one protein. Due to fusion, the two domains are held in very close proximity to each other, one keeping the other stable and in solution (Harada et al., Cancer Res., 2002, 62, 2013-2018).

However, in the design of pharmacological reagents, it is often disadvantageous to create fusion proteins that require a linker sequence to stabilize them.

Australian patent application published AU 2008 202 293 A1 relates to methods of introducing one or more cysteine residues into a polypeptide which permit the stabilization of the polypeptide by formation of a disulfide bond between different domains of the polypeptide. The disclosure also relates to polypeptides containing such introduced cysteine residue(s), nucleic acids encoding such polypeptides and pharmaceutical compositions comprising such polypeptides or nucleic acids

Disulfide bonds are, however, unstable under many physiological conditions. Physiological conditions vary widely, for instance with respect to redox potential (oxidizing vs. reducing) and acidity (high *vs.* low pH) of the various physiological milieus (intracellular, extracellular, pinocytosis vesicles, gastro-intestinal lumen, *etc.).* Disulfide bonds are known to break in reducing environments, such as the intracellular milieu. But even in the extracellular milieu, engineered disulfide bonds are often unstable.

Several other chemical cross-link methodologies allow the formation of bonds that are stable under a broad range of physiological and non-physiological pH and redox conditions. However, in order to maintain the complex's activity and specificity, it is necessary that the cross-link is specifically directed and controlled such that, first, the overall structure of the protein is minimally disrupted, and second, that the cross-link is buried in the protein complex so as not to be immunogenic. However with most cross-link methodologies, the degree to which a bond can be directed to a specific site is too limited to allow them to be used for most bio-pharmaceutical and/or diagnostic applications.

Examples of such cross-link methodologies include UV-cross-linking, and treatment of protein with formamide or glutaraldehyde.

Immunoglobulin Fv fragments comprise another example of a class of proteins for which stabilization is desirable. Immunoglobulin Fv fragments are the smallest fragments of immunoglobulin complexes shown to bind antigen. Fv fragments consist of the variable regions of immunoglobulin heavy and light chains and have broad applicability in pharmaceutical and industrial settings.

To date, a variety of methodologies have been employed to stabilize engineered antibodies. First, introduction of additional disulfide bonds has been performed through molecular biological manipulation of the antibody-expressing construct, without however resolving all the above mentioned drawbacks regarding the use of disulfide bonds. Second, introduction of a linker has been employed that allows both fragments to be expressed as a single chain. Yet, linkers result in rigid conjugates that elicit immune responses, hampering the utility. Linkers that are not immunogenic are generally the more flexible linkers that provide insufficient stability. Finally, fusion of an exogenous di- or oligomerization domain to each of the Fv fragment chains has been performed. Unfortunately, it appears that Fv fragments stabilized by fusion to multimerization domains are significantly immunogenic, and lack the most significant advantage of Fv fragments in the first place: reduced size and resultant increased tissue penetration.

Another approach could be the oxidative cross-link reaction between tyrosyl side-chains, which has been demonstrated to occur naturally, for example in the cytochrome c peroxidase compound I.

The reaction only occurs with tyrosine side-chains that are in very close proximity to each other. Furthermore, the bond formed between the tyrosyl side-chains is irreversible and stable under a very wide range of physiological conditions. Furthermore, the use of dityrosyl cross-linking for formation of buried chemical cross-links for stabilizing a protein complex while maintaining its activities and specificities have not been described in a commercial setting.

International patent application published WO 2015/013551 A1 describes constructs to stabilize or "lock" the respiratory syncytial virus (RSV) F protein in its pre-fusion conformation. The RSV F protein is known to induce potent neutralizing antibodies that correlate with protection against the virus. This disclosure provides RSV F polypeptides, proteins, and protein complexes, such as those that can be or are stabilized or "locked" in a pre-fusion conformation, for example using targeted cross-links, such as targeted di-tyrosine cross-links. This disclosure also provides specific locations within the amino acid sequence of the RSV F protein at which, or between which, cross-links can be made in order to stabilize the RSV F protein in its pre-F conformation. Where di-tyrosine crosslinks are used, the disclosure provides specific amino acid residues (or pairs of amino acid residues) that either comprise a pre-existing tyrosine residue or can be or are mutated to a tyrosine residue such that di-tyrosine cross-links can be made.

In the search to produce proteins with an increased stability, researchers have demonstrated in US patent application published US 2012/0141423 A1 that modified residues comprising notably dehydrated amino acids, i.e., α,β-didehydroaianine (Dha) and α,β-didehydrobutyric acid (Dhb) and thioether bridges of the nonproteinogenic amino acid lanthionine, can stabilize molecular conformations that are essential for the antimicrobial activity of antimicrobial peptide (AMP). An example of AMP which is stabilized with dehydrated amino acids residue is nisin, a lantibiotic approved by the World Health Organization as a food preservative. Other works concerning lantibiotic and stabilizing dehydrated amino acids are described in US patent application published US 5,932,469 which relates to bacteriocins, in US patent published US 7,479,781 B2 which relates to compounds and pharmaceutical compositions for the treatment of ocular diseases and disorders, or in US patent published US 8,691,773 B2 which relates to a peptide compound with biological activity, in particular possessing antimicrobial properties.

Didehydro-phenylalanine (ΔPhe) is regarded as being among the best choices to fix the 3D structure of short, often non-ribosomal peptides. However the potential to introduce ΔPhe in proteins produced using a natural production system was previously unknown, hence ΔPhe could only be introduced in intact polypeptides using solid phase protein synthesis or similar chemical techniques as was demonstrated by the introduction of a functional hinge in insulin (Menting et al. PNAS, 2014, E3395-E3404), a production system unsuitable for large-scale production of catalysts for industrial applications.

Prior art paper published Rao *et al.* 2008 discloses the modification of plant proteins and the incorporation of specific sequences that will impart the given 3D structure once the sequence is incorporated.

### Summary of invention

### Technical Problem

The invention has for technical problem to alleviate at least one of the drawbacks present in the prior art.

### Technical solution

The first object of the invention is directed to a method for incorporating a recognition sequence in a protein, said method comprising the steps of (a) generating at least one genetic construct comprising a nucleotide sequence coding for said protein comprising the recognition sequence; (b) expressing in a natural host said at least one genetic construct using a vector comprising said at least one genetic construct; and (c) using a plant-based over-expression system with a constitutive promoter to over-express said vector, said plant-based over-expression system is based on at least one plant and/or on at least one plant cells suspension. Said method is remarkable in that said recognition sequence comprises at least one stretch of amino acids with as sequence Phe-x₁-x₂-Tyr, wherein Phe is phenylalanine, x₁ and x₂ are amino acid residues and Tyr is tyrosine.

In a preferred embodiment, x₁ and x₂ are polar hydroxyl-containing amino acid and/or basic amino acid.

Said plant-based over-expression system has an enzymatic activity which converts the phenylalanine residue of said recognition sequence into a didehydro-phenylalanine residue.

In a preferred embodiment, said plant-based over-expression system is based on at least one plant belonging to the group of rosids, preferentially to the group of fabids or malvids.

In a preferred embodiment, said plant-based over-expression system is based on *Medicago sativa, Arabidopsis thaliana* and/or *Cannabis sativa.*

In a preferred embodiment, said at least one genetic construct comprising said recognition sequence is based on a focus sequence of one beta subunit of polygalacturonase of the *Medicago sativa* represented by SEQ-ID NO:3, wherein said focus sequence is delimited by the residue numbered 190 and by the residue numbered 219.

In a preferred embodiment, the residue numbered 203 is phenylalanine and the residue numbered 206 is phenylalanine.

In a preferred embodiment, the residue numbered 203 is phenylalanine and the residue numbered 206 has been modified from phenylalanine to tyrosine.

In a preferred embodiment, the residue numbered 203 has been modified from phenylalanine to tyrosine and the residue numbered 206 is phenylalanine.

The second object of the present invention is directed to a method for producing a structurally-modified protein, comprising the method in accordance with the first object of the invention and at least one subsequent step of isolation of the protein with the recognition sequence with a didehydro-phenylalanine residue from said plant-based over-expression system.

The third object of the present invention is directed to a structurally-modified lipase or insulin containing the recognition sequence with a didehydro-phenylalanine residue obtainable by the method in accordance of the second object of the present invention.

The fourth object of the present invention is directed to the use of the structurally-modified lipase, in accordance with the third object of the invention, as a biocatalyst.

The fifth object of the present invention is directed to a structurally-modified insulin, in accordance with the third object of the invention, for use as protein therapeutics.

### Advantages of the invention

The enzymatic conversion of a normal phenylalanine residue to a didehydro-form allows to use the entire machinery of cells or organisms to generate recombinant proteins with the desired, stable fold. Compared to other solutions for the technical problem it avoids the low yields for chemically introducing unnatural amino acids in recombinant proteins (and associated high production cost).

### Brief description of drawings

Figure 1: Representation of the MS/MS spectrum of the peptide represented by SEQ-ID NO:1.
Figure 2: Table comprising the peaks of the MS/MS spectrum of figure 1.

### Detailed description

The invention proposes the incorporation of a phenylalanine, or a stretch of amino acids containing a phenylalanine, at critical positions in recombinant proteins, a phenylalanine that after enzymatic modification provides a conformationally-restrained bending point in the 3D structure of the protein.

The conformational determination originates from an enzymatic dehydration of the alpha-beta carbon bond of phenylalanine, making it controllable.

First of all dynamic modelling of the stabilized recombinant protein (hereafter designated as "product") and variations thereof will be done to identify the molecular form with the highest stability while the enzymatic properties of the product are similar or better than that of the wild type protein. The product has to include the determined recognition sequence, including the phenylalanine residue that is to be dehydrated, for the modifying enzyme.

The recognition sequence consists minimally in a phenylalanine residue followed by a tyrosine residue, separated by two other residues, i.e. Phe-x₁-x₂-Tyr with x₁ and x₂ being amino acid residues, dominantly being polar hydroxyl-containing and/or basic amino acids. The tyrosine at the +3 position is likely to be essential for the modification to occur.

It is however not excluded that the recognition sequence may be longer.

SEQ-ID NO:1 is part of the protein sequence of the beta subunit of polygalacturanose (alfalfa contig 53863). This particular part of the protein sequence has been identified thanks to mass spectrometry analysis, in particular tandem mass spectrometry (MS/MS). General information about the whole protein sequence can be retrieved on http://plantgrn.noble.org/AGED/.

In SEQ-ID NO:1, the sequences of interest which are recognized by the modifying enzyme comprised in the natural host are (1) Phe₈₀₀ and Tyr₈₀₃; and (2) Phe₈₁₄ and Tyr₈₁₇.

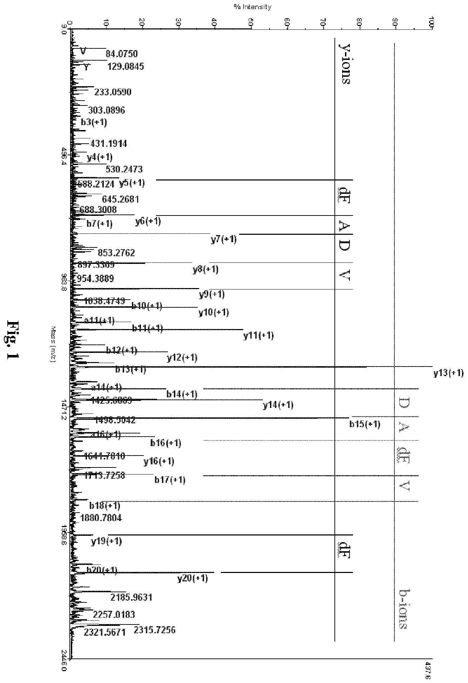

Figure 1 shows the MS/MS spectrum of peptide represented by SEQ-ID NO:1. The dF residues as indicated on the spectrum corresponds to didehydro-phenylalanine (ΔPhe) with a residual mass of 145 Da compared to the residual mass of 147 Da for the unmodified phenylalanine.

Both recognition sequences (1) and (2) have thus been identified. Figure 1 specifically indicates the y-ion (i.e., those fragment peaks that appear to extend from the C-terminus) series as well the b-ion (i.e., those fragment peaks that appear to extend from the N-terminus) series.

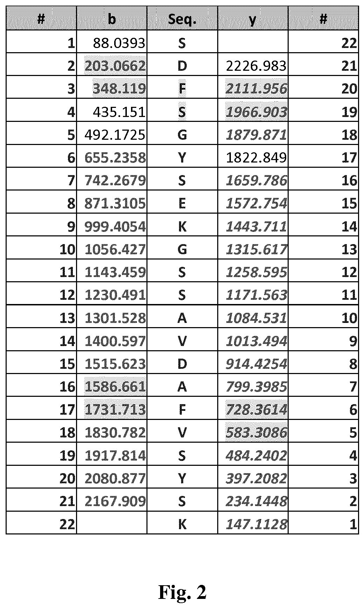

Figure 2 shows a table corresponding to the matching peaks of the MS/MS spectrum given in figure 1. The fragment ions given in b2, b3 and in y20, y19 corresponds to ΔPhe (or dF) from the recognition sequence (1) Phe800 and Tyr803. The fragments ions given in b16, b17 and in y5, y6 corresponds to ΔPhe (or dF) from the recognition sequence (2) Phe814 and Tyr817. It indeed illustrates the 145 Da mass compared to the mass of 147 Da normally expected for an unmodified Phe.

In order to confirm the results obtained by mass spectrometry, the use of the MASCOT software enables the identification of proteins by interpreting mass spectrometry data.

Searching via MASCOT database thus results in a highly significant match between spectrum and the peptide sequence with ΔPhe. The mascot score is of 148 (a score superior to 47 being considered as significant) and an expected value of 9.3e-0.12.

The following experiments, with a coding sequence for the alfalfa beta subunit of polygalacturonase represented by the sequence SEQ-ID NO:2 and SEQ-ID NO:3 (known under the reference alfalfa contig Medtr8g064530) or still by the sequences of alfalfa contig 53863 represented by SEQ-ID NO:4, SEQ-ID NO:5, and SEQ-ID NO:6 have been undertaken to confirm the above results, i.e. the determination of the recognition sequence required for a modifying enzyme to change the conformation of a protein.

General information about the protein sequence alfalfa contig Medtr8g064530 (SEQ-ID NO:2 and SEQ-ID NO:3) can be retrieved on http://plantgrn.noble.org/LegumeIPv2/.

General information about the protein sequence alfalfa contig 53863 (SEQ-ID NO:4, SEQ-ID NO:5 and SEQ-ID NO:6) can be retrieved on http://plantgrn.noble.org/AGED/.

In order to achieve the introduction of the modified amino acid residues in the chain of recombinant proteins different genetic constructs have to be created.

Synthetic constructs of the protein sequence alfalfa contig Medtr8g064530 are overexpressed in a plant cell suspension culture and the effect of site directed mutations of phenylalanine and tyrosine residues on the stabilities are studied.

Site-directed mutants of phenylalanine are generally made by the replacement of the phenylalanine with tyrosine.

The focus sequence is represented in SEQ-ID NO:3.

The advantages of focusing on this sequence provide several benefits, as stated below:

First, it contains the only Phe (Phe₂₀₃) experimentally found to be unmodified when the recognition sequence Phe₂₀₆-x-x-Tyᵣ₂₀₉ has been exploited by the modifying enzyme.

The replacement of Phe₂₀₃ by Tyr indicates that the recognition sequence Phe₂₀₆-x-x-Tyᵣ₂₀₉ is still usable for the modifying enzyme.

Secondly, the replacement of Phe₂₀₆ by Tyr abolishes the modification site at residue 206 (i.e., the recognition sequence Phe₂₀₆-x-x-Tyr₂₀₉ is not anymore usable for the modifying enzyme) but creates the recognition sequence Phe₂₀₃-x-x-Tyr₂₀₆. These studies allow to establish that the sequence Phe-x-x-Tyr is sufficient as recognition sequence. Potentially, other surrounding amino acids are important in the recognition of the site of modification by the modifying enzyme.

The following step is the generation of site directed mutants. Different constructs are made: one construct for the full length native protein, one construct for the full length with Phe₂₀₃ to Tyr, one construct for the full length with Phe₂₀₆ to Tyr, one construct for the full length with Tyr₂₀₉ to Phe and one empty vector.

This protein sequence of the beta subunit of polygalacturanose (alfalfa contig 53863) has been employed to confirm that recognition sequence comprises at least one stretch with as sequence Phe-x₁-x₂-Tyr.

In the SEQ-ID NO:4, the sequences of interest which are recognized by the modifying enzyme are (3) Phe₁₀₂ and Tyr₁₀₅ ; and (4) Phe₁₃₀ and Tyr₁₃₃.

In the SEQ-ID NO:5, the sequences of interest which are recognized by the modifying enzyme are (5) Phe₂₀₀ and Tyr₂₀₃ ; (6) Phe₂₁₄ and Tyr₂₁₇ ; (7) Phe₂₂₉ and Tyr₂₃₂ ; and (8) Phe₂₄₃ and Tyr₂₄₆. It is noted that the residue Phe₂₃₆ is not affected at all by the modifying enzyme.

In the SEQ-ID NO:6, the sequence of interest which is recognized by the modifying enzyme is (9) Phe₂₇₇ and Tyr₂₈₀.

Oligonucleotide primers are generated for the different constructs and are used to generate mutants with the specified amino acid changes. These mutants are generated using according to methods for site directed mutagenesis well known in the art.

These generated vectors are overexpressed in plant-based expression system (*i.e*., plant and/or plant cell suspensions) using a constitutive promotor. Since the modifying enzyme that converts phenylalanine into the didehydro form is inherently present in the plant suspension cell culture, no extra modifications of this production system are required. This is achieved *in situ.*

The modifying enzyme converts phenylalanine into didehydro-phenylalanine, thereby changing the tri-dimensional structure of the natural protein, thereby stabilizing the protein and making it less sensitive to various change of the environment, like the temperature and/or the pH.

The synthesis of the stabilized recombinant protein in a system containing the modifying enzyme which is able to interact actively with the recognition sequence is thus performed. The modifying enzyme is co-expressed in the same natural host (*in situ*) whereby the target phenylalanine is modified prior to the isolation of the product from the appropriate culture system. The used host must also produce the modifying enzyme. For plant species (notably alfalfa, hemp and thale-cress) this activity is naturally present but it may be required to make the modifying activity of the host inducible. For other culture systems no naturally occurring modifying activity is known. In these cases a second construct, containing the modifying enzyme, must be made using the same vector to ensure the concomitant expression of both the product gene and the modifying enzyme.

By this *in situ* approach, the stabilized recombinant protein can be obtained directly.

This process is suitable for the stabilization of natural proteins comprising a large number of amino acids. This process leads in fact to a modified natural protein, the modification being in the tri-dimensional structure of the protein. It is a process for the stabilization and functional customization of proteins through the incorporation of a stable, conformation-determining amino acid in a protein sequence.

Once the modifying enzyme has performed the structural modification, the product can be isolated from the culture medium using a pull-down approach with antibodies or other techniques currently used in the art to isolate recombinant proteins. Analytical techniques known by the skilled person in the art will be also employed to determine the structure and to check the stability of the structurally modified protein. For instance, mass spectrometry analysis, fluorescence testing and ELISA test, among many other, might be used.

The stabilized recombinant protein may be used in different systems as biocatalysts (*e.g*. production of biodiesel by lipases, biomass valorisation, lignin cleavage, *etc*.) or in protein therapeutics (*e.g*. stabilized forms of insulins, stabilized forms of antibodies, *etc*.).
## Claims

1. Method for incorporating a recognition sequence in a protein, said method comprising the steps of
a. generating at least one genetic construct comprising a nucleotide sequence coding for said protein comprising the recognition sequence;
b. expressing in a natural host said at least one genetic construct using a vector comprising said at least one genetic construct; and
c. using a plant-based over-expression system with a constitutive promoter to over-express said vector, said plant-based over-expression system is based on at least one plant and/or on at least one plant cells suspension;
**characterized in that** said recognition sequence comprises at least one stretch of amino acids with as sequence Phe-x₁-x₂-Tyr, wherein Phe is phenylalanine, x₁ and x₂ are amino acid residues and Tyr is tyrosine and **in that** said plant-based over-expression system has an enzymatic activity which converts the phenylalanine residue of said recognition sequence into a didehydro-phenylalanine residue.

2. Method according to claim 1, **characterized in that** said plant-based over-expression system is based on at least one plant belonging to the group of rosids, preferentially to the group of fabids or malvids.

3. Method according to any one of claims 1-2, **characterized in that** said plant-based over-expression system is based on *Medicago sativa, Arabidopsis thaliana* and/or *Cannabis sativa.*

4. Method according to any one of claims 1-3, **characterized in that** x₁ and x₂ are polar hydroxyl-containing amino acid and/or basic amino acid.

5. Method according to any one of claims 1-4, **characterized in that** said at least one genetic construct comprising said recognition sequence is based on a focus sequence of one beta subunit of polygalacturonase of the *Medicago sativa* represented by SEQ-ID NO:3, wherein said focus sequence is delimited by the residue numbered 190 and by the residue numbered 219.

6. Method according to claim 5, **characterized in that** the residue numbered 203 is phenylalanine and the residue numbered 206 is phenylalanine.

7. Method according to claim 5, **characterized in that** the residue numbered 203 is phenylalanine and the residue numbered 206 has been modified from phenylalanine to tyrosine.

8. Method according to claim 5, **characterized in that** the residue numbered 203 has been modified from phenylalanine to tyrosine and the residue numbered 206 is phenylalanine.

9. Method for producing a structurally-modified protein, comprising the method of any one of claims 1-8 and at least one subsequent step of isolation of the protein with the recognition sequence with a didehydro-phenylalanine residue from said plant-based over-expression system.

10. Structurally-modified lipase or insulin containing the recognition sequence with a didehydro-phenylalanine residue obtainable by the method of claim 9.

11. Use of the structurally-modified lipase according to claim 10 as a biocatalyst.

12. Structurally-modified insulin according to claim 10 for use as protein therapeutics.

## Patentansprüche

1. Verfahren zum Einbau einer Erkennungssequenz in ein Protein, wobei das Verfahren die folgenden Schritte umfasst
a. Erzeugen mindestens eines genetischen Konstrukts, das eine Nukleotidsequenz umfasst, die das Protein kodiert, das die Erkennungssequenz umfasst;
b. Exprimieren des mindestens einen genetischen Konstrukts in einem natürlichen Wirt unter Verwendung eines Vektors, der das mindestens eine genetische Konstrukt umfasst; und
c. Verwenden eines auf Pflanzen basierenden Überexpressionssystems mit einem konstitutiven Promotor zur Überexpression des Vektors, wobei das auf Pflanzen basierende Überexpressionssystem auf mindestens einer Pflanze und/oder auf mindestens einer Pflanzenzellensuspension basiert;
**dadurch gekennzeichnet, dass** die Erkennungssequenz mindestens eine Abfolge von Aminosäuren mit einer Sequenz Phe-x₁-x₂-Tyr umfasst, wobei Phe Phenylalanin ist, x₁ und x₂ Aminosäurereste sind und Tyr Tyrosin ist, und dass das pflanzliche Überexpressionssystem eine enzymatische Aktivität aufweist, die den Phenylalaninrest der Erkennungssequenz in einen Didehydro-Phenylalaninrest umwandelt.

2. Verfahren nach Anspruch 1, **dadurch gekennzeichnet, dass** das auf Pflanzen basierende Überexpressionssystem auf mindestens einer Pflanze basiert, die zur Gruppe der Rosiden, vorzugsweise zur Gruppe der Fabiden oder Malviden, gehört.

3. Verfahren nach einem der Ansprüche 1-2, **dadurch gekennzeichnet, dass** das auf Pflanzen basierende Überexpressionssystem auf *Medicago sativa, Arabidopsis thaliana* und/oder *Cannabis sativa* basiert.

4. Verfahren nach einem der Ansprüche 1-3, **dadurch gekennzeichnet, dass** x₁ und x₂ jeweils eine polare hydroxylhaltige Aminosäure und/oder basische Aminosäure sind.

5. Verfahren nach einem der Ansprüche 1-4, **dadurch gekennzeichnet, dass** das mindestens eine genetische Konstrukt, das die Erkennungssequenz umfasst, auf einer fokalen Sequenz einer beta-Untereinheit von Polygalacturonase *von Medicago sativa,* dargestellt durch SEQ-ID Nr. 3, basiert, wobei die fokale Sequenz durch den Rest mit der Nummer 190 und durch den Rest mit der Nummer 219 begrenzt ist.

6. Verfahren nach Anspruch 5, **dadurch gekennzeichnet, dass** der Rest mit der Nummer 203 Phenylalanin ist und der Rest mit der Nummer 206 Phenylalanin ist.

7. Verfahren nach Anspruch 5, **dadurch gekennzeichnet, dass** der Rest mit der Nummer 203 Phenylalanin ist und der Rest mit der Nummer 206 von Phenylalanin zu Tyrosin modifiziert wurde.

8. Verfahren nach Anspruch 5, **dadurch gekennzeichnet, dass** der Rest mit der Nummer 203 von Phenylalanin zu Tyrosin modifiziert wurde und der Rest mit der Nummer 206 Phenylalanin ist.

9. Verfahren zur Herstellung eines strukturell modifizierten Proteins, umfassend das Verfahren nach einem der Ansprüche 1-8 und mindestens einen nachfolgenden Schritt der Isolierung des Proteins mit der Erkennungssequenz mit einem Didehydro-Phenylalanin-Rest aus dem pflanzenbasierten Überexpressionssystem.

10. Strukturell modifizierte Lipase oder Insulin, die Erkennungssequenz mit einem Didehydro-Phenylalanin-Rest enthaltend, der nach dem Verfahren von Anspruch 9 erhalten werden kann.

11. Verwendung der strukturell modifizierten Lipase nach Anspruch 10 als Biokatalysator.

12. Strukturell modifiziertes Insulin nach Anspruch 10 zur Verwendung als Proteintherapeutikum.

## Revendications

1. Procédé destiné à l'incorporation d'une séquence de reconnaissance dans une protéine, ledit procédé comprenant les étapes dans lesquelles :
a. on génère au moins une construction génétique qui comprend une séquence nucléotidique codant pour ladite protéine qui comprend la séquence de reconnaissance ;
b. on exprime dans un hôte naturel ladite au moins une construction génétique en utilisant un vecteur qui comprend ladite au moins une construction génétique ; et
c. on utilise un système de surexpression à base de plante qui comprend un promoteur constitutif afin de surexprimer ledit vecteur, ledit système de surexpression à base de plante se basant sur au moins une plante et/ou sur au moins une suspension de cellules végétales ;
**caractérisé en ce que** ladite séquence de reconnaissance comprend au moins un segment d'acides aminés comprenant, à titre de séquence, Phe-x₁-x₂-Tyr, dans laquelle Phe représente la phénylalanine, x₁ et x₂ représentent des résidus d'acides aminés et Tyr représente la tyrosine ; et **en ce que** ledit système de surexpression à base de plante possède une activité enzymatique qui transforme le résidu de phénylalanine de ladite séquence de reconnaissance en un résidu de didéshydro-phénylalanine.

2. Procédé selon la revendication 1, **caractérisé en ce que** ledit système de surexpression à base de plante se base sur au moins une plante qui appartient au groupe des rosidées, de manière préférentielle au groupe des fabidées ou des malvidées.

3. Procédé selon l'une quelconque des revendications 1 à 2, **caractérisé en ce que** ledit système de surexpression à base de plante est à base de *Medicago sativa, d'Arabidopsis thaliana* et/ou de *Cannabis sativa.*

4. Procédé selon l'une quelconque des revendications 1 à 3, **caractérisé en ce que** x₁ et x₂ représentent des acides aminés polaires contenant un groupe hydroxyle et/ou des acides aminés basiques.

5. Procédé selon l'une quelconque des revendications 1 à 4, **caractérisé en ce que** ladite au moins une construction génétique qui comprend ladite séquence de reconnaissance se base sur une séquence d'intérêt d'une sous-unité bêta de la polygalacturonase de Medicago *sativa,* représentée par la SEQ ID NO: 3 ; dans lequel ladite séquence d'intérêt est délimitée par le résidu portant le numéro 190 et par le résidu portant le numéro 219.

6. Procédé selon la revendication 5, **caractérisé en ce que** le résidu portant le numéro 203 représente la phénylalanine et le résidu portant le numéro 206 représente la phénylalanine.

7. Procédé selon la revendication 5, **caractérisé en ce que** le résidu portant le numéro 203 représente la phénylalanine et le résidu portant le numéro 206 a été modifié pour passer de la phénylalanine à la tyrosine.

8. Procédé selon la revendication 5, **caractérisé en ce que** le résidu portant le numéro 203 a été modifié pour passer de la phénylalanine à la tyrosine et le résidu portant le numéro 206 représente la phénylalanine.

9. Procédé destiné à la production d'une protéine dont la structure a été modifiée, comprenant le procédé selon l'une quelconque des revendications 1 à 8 et au moins une étape ultérieure d'isolation de la protéine comprenant la séquence de reconnaissance qui comprend un résidu de didéshydro-phénylalanine, à partir dudit système de surexpression à base de plante.

10. Insuline ou lipase dont la structure a été modifiée contenant la séquence de reconnaissance qui comprend un résidu de didéshydro-phénylalanine que l'on peut obtenir par l'intermédiaire du procédé selon la revendication 9.

11. Utilisation de la lipase dont la structure a été modifiée, selon la revendication 10, à titre de biocatalyseur.

12. Insuline dont la structure a été modifiée, selon la revendication 10, pour son utilisation à titre d'agent thérapeutique à base de protéine.


<110> LIST
   Sergeant Kjell
<120> Method for enzymatically modifying the tri-dimensional structure of a protein
<130> sequence listing of PT04271 WO
<160> 6
<210> 1
   <211> 22
   <212> PRT
   <213> Alfalfa contig 53863 (part of)
<223> Beta subunit of polygalacturonase
<400> 1 
Ser Asp Phe Ser Gly Tyr Ser Glu Lys Gly Ser Ser Ala Val Asp Ala Phe Val Ser
Tyr
798 802 807 812
817
Ser Lys
818
<210> 2
   <211> 644
   <212> PRT
   <213> Alfalfa contig Medtr8g064530
<223> Beta subunit of polygalacturonase
<400> 2 
Met Lys Lys Met Gln Lys Gln Leu Gly His Ile Ile His Thr Leu Phe Ile Leu Leu
Ser
1               5                   10                  15
20
Ile Leu Thr Val Gly Leu Ala Ile Asn His Glu Lys Glu Lys Asn Gln Asn Pro Phe
Thr
21              25                  30                  35
40
Pro Lys Ala Tyr Val Ile Arg Tyr Trp Asp Arg Val Ile Lys Asn Asn Leu Pro Lys
Pro
41              45                  50                  55
60
Ser Phe Ile Leu Ser Lys Ala Ser Pro Leu Ser Ala Thr Glu Ala Ala Thr Phe Thr
Lys
61              65                  70                  75
80
His Ala Ala Ala Asn Thr Leu Ser Thr Lys Leu Pro Glu Phe Cys Ser Ala Ala His
Leu
81              85                  90                  95
100
Phe Cys Phe Pro Glu Leu Thr Lys Gln Arg Pro Phe Asp Pro Ser His Phe Thr Val
Tyr
101             105                 110                 115
120
Glu Thr Asp Gln Asn Phe Thr Phe Tyr Ser Leu Gly Glu Asn Phe Thr Thr Tyr Gly
 
Thr
121                 125                 130                 135
140
His Lys Leu Asn Gly Ile Asp Ala Phe Lys Asn Tyr Ser Asn Gly Leu Tyr Asn Glu
Lys
141                 145                 150                 155
160
His Asn Glu Phe Ala Arg Tyr Ser Lys Ile Ser Thr Gly His Asn Asp Ser Phe Lys
Asn
161                 165                 170                 175
180
Tyr Ala Leu Asn Asn Asn Glu Gly Val Gln Thr Phe Asn Ser Tyr Gly Thr Lys Ser
Met
181                 185                 190                 195
200
Ala Gly Phe Gly Glu Phe Lys Ala Tyr Ser Glu Glu Ser Asn Ile Pro Asn Leu Lys
Phe
201                 205                 210                 215
220
Thr Thr Tyr Thr Ala Ser Val Thr Gly Arg Glu Gln Ser Phe Val Ser Tyr Ser Asp
Gly
221                 225                 230                 235
240
Gly Asn Ser Gly Asn Gln Ser Phe Ala Ser Tyr Gly Lys Asp Ser Ile His Ala Val
Asn
241                 245                 250                 255
260
Gly Phe Thr Gln Tyr Ala Lys Asn Ala Asn Val Phe Lys Ser Asp Phe Ser Gly Tyr
Ser
261                 265                 270                 275
280
Glu Lys Gly Ser Ser Ala Val Asp Ala Phe Val Ser Tyr Ser Lys Asn Leu Asn Lys
Pro
281                 285                 290                 295
300
Glu Asn Thr Phe Lys Asn Tyr Gly Lys Gly Ser Phe Ala Ala Gln Glu Lys Phe Thr
Asn
301                 305                 310                 315
320
Tyr Arg Asp Gly Ala Gly Ser Gly Asp Asp Ser Phe Thr Ser Tyr Gly Glu Asn Ser
Gln
321                 325                 330                 335
340
Arg Gly Val Lys Val Asp Phe Asn Asn Tyr Gly Gln Asn Pro Asp Tyr Pro Lys Ser
Pro
341                 345                 350                 355
360
Gly Ser Glu Thr Phe Lys Gly Tyr Ala Lys Gly Ala Asp Leu Asp His Lys Val Ser
Phe
361                 365                 370                 375
380
Lys Val Tyr Asn Gly Asn Asn Asn Thr Phe Lys Asp Tyr Asp Lys Lys Gly Ile Ser
Phe
381                 385                 390                 395
400
Ala Ser Tyr Asn Lys Thr Thr Thr Ser Thr Asn Ser Leu Ser Asp Ser Gly Ser Leu
Ala
401                 405                 410                 415
420
Lys Asn Met Val Gln Pro Gly Lys Phe Phe Arg Glu Lys Met Leu Lys Glu Gly Thr
Val
421                 425                 430                 435
440
 
Met Pro Met Pro Asp Ile Arg Asp Lys Leu Pro Pro Arg Ser Phe Leu Pro Arg Ser
Ile
441                 445                 450                 455
460
Leu Thr Lys Leu Pro Phe Ala Ser Ser Lys Leu Asn Glu Met Lys Gln Val Phe Lys
Val
461                 465                 470                 475
480
Ser Glu Asn Ser Ser Met Asn Lys Met Met Val Asp Ala Leu Ser Glu Cys Glu Arg
Ala
481                 485                 490                 495
500
Pro Ser Val Gly Glu Thr Lys Arg Cys Val Gly Ser Leu Glu Asp Met Ile Asp Phe
Ala
501                 505                 510                 515
520
Thr Ser Val Leu Gly His Ser Val Thr Val Arg Ser Thr Glu Asn Val Asn Gly Ala
Gly
521                 525                 530                 535
540
Lys Asp Val Met Val Gly Lys Val Lys Gly Ile Asn Gly Gly Lys Val Thr Glu Ser
Val
541                 545                 550                 555
560
Ser Cys His Gln Ser Leu Phe Pro Tyr Leu Leu Tyr Tyr Cys His Ser Val Pro Asn
Val
561                 565                 570                 575
580
His Val Tyr Glu Ala Asp Leu Leu Asp Pro Val Ser Lys Ala Lys Ile Asn His Gly
Val
581                 585                 590                 595
600
Ala Ile Cys His Leu Asp Thr Thr Ala Trp Ser Ser Thr His Gly Ala Phe Met Ala
Leu
601                 605                 610                 615
620
Gly Ser Ala Pro Gly Gln Ile Glu Val Cys His Trp Ile Phe Glu Asn Asp Met Thr
Trp
621                 625                 630                 635
640
Ser Ser Ala Asp
641
<210> 3
   <211> 30
   <212> PRT
   <213> Alfalfa contig Medtr8g064530 (part of)
<223> Focus sequence
<400> 3 
Gln Thr Phe Asn Ser Tyr Gly Thr Lys Ser Met Ala Gly Phe Gly Glu Phe Lys Ala
Tyr
190                 194                     199                     204
209
Ser Glu Glu Ser Asn Ile Pro Asn Leu Lys
210                 214                     219
<210> 4
   <211> 42
   <212> PRT
   <213> Alfalfa contig 53863 (part of)
<223> Beta subunit of polygalacturonase
<400> 4 
Asp Thr Asn Val Pro Asn Leu Arg Phe Thr Thr Tyr Ser Val Gly Val Ala Gly Arg
Gln
94 98 103 108
113
Gln Thr Phe Thr Ser Tyr Ser Glu Ala Gly Asn Ala Gly Asp Gln Ser Phe Gly Asn
Tyr
114 118 123 128
133
Gly Lys
134
<210> 5
   <211> 57
   <212> PRT
   <213> Alfalfa contig 53863 (part of)
<223> Beta subunit of polygalacturonase
<400> 5 
Gly Ser Leu Gly Ala Val Glu Lys Phe Ser Asn Tyr Arg Asp Gln Ala Asn Val Gly
Ala
192                 196                     201                     206
211
Asp Ser Phe Ser Ser Tyr Ala Lys Asp Ser Thr Gly Gly Thr His Val Asp Phe Asp
Asn
212                 216                     221                     226
231
Tyr Gly Lys Ser Phe Asn Glu Gly Thr Asp Ser Phe Lys Gly Tyr Ala Lys
232                 236                     241                     246
<210> 6
   <211> 10
   <212> PRT
   <213> Alfalfa contig 53863 (part of)
<223> Beta subunit of polygalacturonase
<400> 6

Lys Gly Ile Ser Phe Ala Gly Tyr Thr Lys
273             277                 282